# EUROPEAN PATENT SPECIFICATION

(11) **EP 1 809 345 B1**
(45) Date of publication and mention of the grant of the patent: **18.03.2009**
(21) Application number: 05715151.6
(22) Date of filing: 06.04.2005
(51) Int. Cl.: A61L 29/08, A61L 29/14, C09D 175/04, C09D 171/02

(54) **A MEDICAL DEVICE HAVING A WETTED HYDROPHILIC COATING**
MEDIZINISCHE VORRICHTUNG MIT EINER BEFEUCHTETEN HYDROPHILEN BESCHICHTUNG
DISPOSITIF MEDICAL A REVETEMENT HYDROPHILE HUMIDIFIE

(30) Priority: 07.10.2004 WO PCT/DK2004/000677
(43) Date of publication of application: 25.07.2007
(73) Proprietor: COLOPLAST A/S, 3050 Humlebaek (DK)
(72) Inventor: MADSEN, Niels, Joergen, DK-3450 Alleroed (DK); SCHOENFELDT, Lars, DK-3000 Helsingoer (DK)
(86) International application number: PCT/DK2005/000233
(87) International publication number: WO 2006/037321

(56) References cited:
- EP-A- 0 439 908

## Description

The present invention relates to a medical device having a wetted hydrophilic coating providing a low friction surface to the medical device. In particular, the present invention relates to a wetted medical device, which may be packed and stored in a ready-to-use form, i.e. wetted and ready to insert into a body cavity without having to wet the coating before insertion. The medical devices of the invention have a reduced tendency to leave coating material on the surfaces which it has been in contact with. Furthermore, the wetted medical devices of the invention has an extended dry-out period and they do not drip or have a reduced tendency to drip.

### Background

Medical devices for insertion into a body cavity, such as catheters, guide wires, wound drains and fibre optical articles having a hydrophilic coating providing a low friction surface are known.

In connection with catheters, the presence of a friction reducing hydrophilic coating causes the surface of the catheter to become slippery and lubricating when the catheter is dipped into an aqueous, optionally saline solution prior to insertion into a body cavity. Thus, the discomfort experienced by the patient when the catheter is inserted into and removed from the body cavity is considerably reduced. The risk of damaging sensitive tissue in connection with the use of the catheter is at the same time considerably reduced.

The literature discloses several examples of medical devices having hydrophilic coatings, which are stored in dry condition and wetted immediately before use, e.g. insertion into a body cavity (see e.g. US 5.416.131). The literature also discloses medical devices having a hydrophilic coating, which are stored in wetted ready-to-use condition (see e.g. WO 00/30696).

From US 5.416.131 it is known that various water soluble osmolality increasing compounds (e.g. inorganic salts, such as sodium chloride, potassium chloride, potassium iodide, potassium nitrate, organic salts such as sodium citrate or sodium benzoate, mono or disaccharides, such as glucose, sugar alcohols such as sorbitol and glycerol) may be added to the coating in order to increase the osmolality of the wetted coating and prevent the coating from drying out (thereby leading to an increased coefficient of friction) after it has been inserted into a body cavity and comes into contact with body fluids, see e.g. US 5.416.13'1. It has thus been suggested to introduce glycerol into a hydrophilic coating in order to achieve an osmolality increasing effect.

WO 00/030696 suggest the use of glycerol and diethylene glycol as a plastiziser by adding these substances to the wetting agent.

There is no guidance in this reference as to the amount of plastiziser added to the wetting agent, nor is it described that a ready-to-use device which does not drip and which has a reduced tendency to leave coating material on the surfaces it comes into contact with, may be achieved by wetting the coating with a wetting agent comprising glycerol or diethylene glycol.

These known, devices have a serious drawback in that they need an excess amount of water and have a wet slimy surface, which leads to "smudging" of the body cavities in which they are inserted. Further, these types of medical devices may be dripping in wetted condition and may, depending on the content of wetting agent and the coating material, and stain the clothes of the user.

In US 5.800.412, glycerol is used as a solvent for the coating solution.

In US 5.091.205 it is mentioned that conventional catheters may be rendered lubricant by coating with a layer of silicone fluid, glycerol, or olive oil. This way of lubricating a catheter perform poorly in practice as the lubricant is not eve nly distributed and tend to run off quickly. Another problem is smudging during handling and use of such catheters.

Thus, there is a need for medical devices having a hydrophilic coating, which may be packed and stored in a wetted ready-to-use form, which do not drip and which have a reduced tendency to smudging, i.e. leaving coating material on the surfaces they have been in contact with, e.g. in the body cavities in which they have been inserted.

It has now surprisingly been found that this may be achieved by using a wetting agent comprising mixture of water and a lubricant, such as for example glycerol or ethylene glycol, for wetting of a hydrophilic coating.

The medical devices of the invention also have an extended dry-out period, meaning that the devices may be left in the air or in a body cavity for an extended period of time, without loss of the low friction properties of the coating.

### Summary of the Invention

Thus, in its broadest aspect, the present invention relates to a medical device having a wetted hydrophilic coating comprising
a) a coating composition containing a hydrophilic polymer and
b) a wetting agent comprising water and one or more lubricant(s).

In a particular embodiment of the invention, the above mentioned hydrophilic polymer is an amphiphilic block copolymer containing hydrophilic and hydrophobic blocks forming a physically cross-linked matrix in the coating composition.

The invention also relates to a method for the preparation of a medical device having a wetted hydrophilic coating and medical devices obtainable by said method.

### Detailed Description of the Present Invention

As used herein the phrase "a medical device having wetted hydrophilic coating" means a medical device, which is wetted and ready-to-use, e.g. ready for insertion into a body cavity.

The term "medical device" should be interpreted in a fairly broad sense. Suitable examples of medical devices (including instruments) are catheters (such as urinary catheters), endoscopes, laryngoscopes, tubes for feeding, tubes for drainage, quide wires, condoms, urishealts, barrier coatings e.g. for gloves, stents and other implants, extra corporeal blood conduits, membranes e.g for dialysis, blood filters, devices for circulatory assistance, dressings for wound care, urinary bags, and ostomy bags. Typically the medical device is selected from catheters, endoscopes, laryngoscopes, tubes for feeding, tubes for drainage, quide wires, stents and other implants.

As used herein physical cross-linked means that polymer molecules are bound together by reversible cross-links based on segregation of domains caused by secondary interactions between the polymer chains. These secondary interactions include hydrogen bonding and ionic bonding, but not covalent bonding.

Unlike covalent cross-links, physical cross-links may be broken when heated and established again upon cooling. This allows the material to be processed, and ' recycled. Coating compositions held together by physical cross-linking are therefore thermoplastic.

As used herein "compatible" means the ability for two or more molecules to homogenously associate in a single phase.

As used herein "incompatible" means the inability of two or more molecules to homogenously associate in a single phase.

As used herein the term wetting agent covers an aqueous solution or emulsion comprising one or more lubricants.

As used herein the term coating composition means the composition making up the coating on the medical device, i.e. not including the solvent(s), which may have been used as a solvent in connection with application of the coating composition to the medical device, and not including the wetting agent comprising one or more lubricants.

In one embodiment of the invention, the medical device or the part of the medical device carrying the hydrophilic coating and the coating composition is made from different materials or different polymer compositions.

According to this embodiment of the invention, the medical device or the part of the medical device having a hydrophilic coating may be formed from any material conventionally used for making such medical devices, see below, and the coating composition is thereafter applied to the medical device.

In another embodiment of the invention, the entire medical device or the part of the medical device having a hydrophilic coating is made of the coating composition.

According to this embodiment of the invention, there is no separate step for application of a coating composition to the surface of the medical device or a part of the surface of the medical device.

The lubricant contained in the wetting agent may be a water-soluble or a water insoluble compound having lubricating properties. Suitably, the lubricant is water soluble and suitably the lubricant also has humectant or hygroscopic properties.

The lubricant may thus be selected from hydrophilic lubricants such as glycerol, glycols, such as ethylene glycol, diethylene glycol, triethylenglycol, propylene glycol, dipropylene glycol, 1,5-pentanediol, block polymers of polyoxyethylenepolyoxypropylene (Polyoxamer), such as Poloxamer 188, Poloxamer 237, Poloxamer 338, Poloxamer 407, Poloxamer 124 from BASF, Polyethylenglycol such as PEG 300, PEG 400, PEG 600 PEG 800, PEG 1000. PEG 1500, PEG 2000, PEG 3000, PEG 3350, PEG 4000, PEG 5500, PEG 6000, PEG 8000, PEG 10000, PEG 12000, PEG 20000 and PEG 35000, statistical Ethylene Oxide / Propylene Oxide-Copolymerisates, such as the Polyglykol P41-types from Clariant, statistical Ethylene Oxide / Propylene Oxide-Copolymerisate-Monobutyl Ethers, such as the Polyglykol B11-types from Clariant, polyalkylene Glycols such as the Polyglykol PR-types (e.g. Polyglykol PR 300, - PR 450, - PR600 and PR 1000) and Glycol esters.

The lubricant(s) may also be selected from hydrophobic lubricants selected from PO Polymerisate-Monobutyl Ethers / PO Polymerisate-Monoisotridecyl Ethers such as the Polyglykol B01 / T01 and the like from Clariant, polypropylene Glycols such as PPG100 - 5000, perfluorethers such as Fomblin PFPE Y, M or W type lubricants from Solvay, paraffinic oil, or silicone oil.

Preferably, the lubricant(s) are selected from hydrophilic lubricants such as glycerol, glycols, such as ethylene glycol, diethylene glycol, triethylenglycol, propylene glycol, dipropylene glycol, 1,5-pentanediol and glycol esters, such as glycerol diacetate (diacetin) and glycerol triacetate (triacetin). Suitably, the wetting agent comprises one lubricant and suitably the lubricant is glycerol.

The hydrophilic polymer contained in the coating composition on the medical device could suitably be selected from poly (N-vinyllactam)s, such as polyvinyl pyrrolidone (PVP), homopolymers of N-vinyl butyrolactam and N-vinyl caprolactam, or copolymers comprising N-vinyl pyrrolidone, vinyl butyrolactam and/or N-vinyl caprolactam, poly(2-ethyl-2-oxazoline), polyvinyl alcohol and derivatives thereof, polysaccharides, dextran, xanthan, hydroxypropyl cellulose, methyl cellulose, hydrophilic polyurethanes, polyhydroxyacrylates, polyhydroxyethylmethacrylate, polyacrylic acid, copolymers of vinyl compounds, e.g. with hydroxyacrylates, such as hydroxyethylmethylacrylate, polyethers, such as polyethylene oxide, copolymers of methyl vinyl ether and maleic acid anhydride, copolymer of maleic acid and styrene.

Some of these hydrophilic polymers may be treated to establish covalent bonds between the polymer chains after the coating composition has been applied to the medical device, see e.g. WO 00/030696.

Preferably, the hydrophilic polymer is a polymer forming a physical cross-linked matrix in the coating composition, e.g. an amphiphilic block copolymer comprising hydrophilic and hydrophobic blocks. The hydrophilic coating is suitably formed from a thermoplastic coating composition comprising an amphiphilic block copolymer forming a physically cross-linked matrix in the coating compositions

In one embodiment of the invention the amphiphilic block copolymer is a hydrophilic polyurethane. Suitable amphiphilic polyurethanes for the purpose of the present invention are ESTANE® T5410 from NOVEON® (B.F.Goodrich), Tecophilic HP93A100, various types of Tecogeis from Thermedics Polymer Products (e.g. Tecogel 500 and Tecogel 2000), HydroMed D640 and HydroSlip from CardioTech international Inc.

In one specific embodiment of the invention, the amphiphilic polyurethane is Tecogel 2000 or Tecophilic HP93A100.

The amphiphilic block copolymers forming the physically cross-linked matrix in the coating composition suitably consist of at least one non-poiar polymeric chain (hydrophobic block) covalently linked to at least one polar polymeric chain (hydrophilic block). The polar chain end of the polymer must be water-soluble or water swellable and take up 200-250 %, suitably least a content of 300 % w/w, preferably at least 500 % w/w of water based on dry matter, if taken alone. The non-polar chain preferably does not take up more than 10 % w/w of water based on dry matter when submersed in water.

The amphiphilic block co-polymers may for instance be a diblock polymer having a structure AB, where A is a hydrophobic block and B is a hydrophilic block or a triblock having a linear structure ABA where A is a hydrophobic block and B is a hydrophilic block, or alternatively have the form of a multi block, or multi arm star-shaped copolymer structure, containing A and B blocks. Suitably, the amphiphilic block co-polymer is a diblock AB or a triblock ABA, and most preferred the amphiphilic block co-polymer is a triblock copolymer ABA.

The monomer units polymerised to form the hydrophobic blocks may be identical or essentially identical or they may be different, e.g. as in a copolymer. The same applies to the hydrophilic blocks. The invention cover the use of all possible combinations of hydrophobic and hydrophilic blocks whether the blocks are homopolymers prepared from essentially identical monomer units or copolymers.

Hydrophobic monomers for preparing the hydrophobic A block are suitably monovinyl aromatic or hetero-aromatic monomers which typically contain from 8 to 18 carbon atoms, such as styrene, alpha-methylstyrene, vinyltoluene, vinylpyridine, ethylstyrene, t-butylstyrene, isopropylstyrene, dimethylstyrene, and other alkylated styrenes.

The styrene monomers in an A block of polystyrene may partly be derived from derivatives of styrene, such as alpha-methylstyrene or vinyltoluene.

Alternatively, the hydrophobic A block may be prepared from ethylenically unsaturated monomers chosen from butadiene, chloroprene, (meth)acrylic esters, vinyl esters such as vinyl acetate, vinyl versatate and vinyl propionate; or vinyl halides such as vinyl chloride, and vinyl nitriles.

"(Meth)acrylic esters" is used in the present context to designate esters of acrylic acid and of methacrylic acid with optionally halogenated, e.g. chlorinated or fluorinated, C₁ -C₃₀ straight or branched alcohols, preferably C₁ -C₁₈ alcohols. Examples of such esters are methyl acrylate, ethyl acrylate, propyl acrylate, n-butyl acrylate, isobutyl acrylate, 2-ethylhexyl acrylate, lauryl acrylate, tert.butyl acrylate, methyl methacrylate, ethyl methacrylate, n-butyl methacrylate and isobutyl methacrylate.

Suitable vinyl nitriles are those having from 3 to 12 carbon atoms, such as, in particular, acrylonitrile and methacrylonitrile.

Alternatively, the hydrophobic A block(s) of the amphiphilic block copolymer is a a poly alpha-olefin, such as polyethylene, polypropylene, poly-1-butene or polyisobutylene

The hydrophobic A block(s) of the amphiphilic block copolymer may also be a polyvinylether, a polyacetate, a polysiloxane, a hydrophobic polyester or similar polymers.

Suitable, the hydrophobic A block(s) of the amphiphilic polymer comprises polymerised styrene, i.e. the hydrophobic block consists essentially of polymerised styrene or the block consists of a styrene monomer copolymerised with other monomers which are hydrophobic in polymerised form.

In one embodiment, the hydrophobic A block(s) of the amphiphilic polymer comprises polymerised acrylic acid ester monomers, i.e. the block(s) consists of polymerised (meth)acrylic acid ester or the block(s) consists of copolymerised (meth)acrylic acid ester monomers and other monomers which are hydrophobic in polymerised form.

In a further embodiment of the invention, the hydrophobic A block(s) of the amphiphilic polymer comprises hydrophobic blocks of a polymerised vinylic unsaturated aliphatic hydrocarbon monomer comprising from 1 to 6 carbon atoms. Preferred and commercially readily available are polymerised vinylic unsaturated hydrocarbon monomers, comprising 4 carbon atoms, polybutylene and polyisobutylene being most preferred.

The most preferred hydrophobic A blocks are blocks of polystyrene, polymethylmethacrylate, polybutylacrylate and poly-2-ethylhexylacrylate.

In one embodiment, the amphiphilic block copolymer comprises different hydrophobic blocks A , e.g. a block of polystyrene and a block of a hydrophobic polyacrylic acid ester.

The coating composition may contain a plasticiser for the hydrophoblic block of the amphiphilic block copolymer.

Suitable hydrophobic plasticizers are e.g. camphor, castor oil, dibutyl phthalate, dibutyl sebacate, dioctyl adipate (DOA), dioctyl adipate (DOP), acetyl tributyl citrate (Citrofol BII), santicizer 141, Santicizer 148, Santicizer 261, Sebacic acid, and Tributyl citrate (Citrofol BI).

The hydrophilic B block(s) of the amphiphilic block copolymer may be any type of hydrophilic polymer that is able to absorb significant amounts of water.

Monomers useful for preparing the hydrophilic B block are for example ethylenically unsaturated monocarboxylic and dicarboxylic acids, such as acrylic acid, methacrylic acid, itaconic acid, maleic acid and fumaric acid; and monoalkyl esters of dicarboxylic acids of the above mentioned type with alkanols, preferably alkanols having from 1 to 4 carbon atoms; N-substituted derivatives (amides) of the above mentioned dicarboxylic acids, amides of unsaturated mono carboxylic acids, such as acrylamide, methacrylamide, N-methoxyacrylamide or methacrylamide, and N-alkylacrylamides; ethylenic monomers containing a sulphonic acid group and ammonium or alkali metal salts thereof, for example vinylsulphonic acid, vinylbenzenesulphonic acid, alpha-acrylamidomethylpropanesulphonic acid and 2-sulphoethylene methacrylate; amides of vinylamine, especially vinylformamide or vinylacetamide; and unsaturated ethylenic monomers containing a secondary, tertiary or quaternary amino group, or a heterocyclic group containing nitrogen, such as, for example, vinylpyridines, vinylimidazole, aminoalkyl (meth)acrylates and aminoalkyl (meth)acrylamides such as dimethylaminoethyl acrylate or methacrylate, di-tert.butylaminoethyl acrylate or methacrylate and dimethylaminoacrylamide or dimethylaminomethacrylamide. It is also possible to use zwitterionic monomers such as, for example, sulphopropyl(dimethyl)-aminopropyl acrylate.

Suitable, the hydrophilic B block(s) in the amphiphilic block copolymers are PEG (polyethylene glycol), poly ethylene oxide, PVP (polyvinyl pyrrolidone), polyacrylic acid, salts of polyacrylic acid, salts of polymers of composed of acids such as maleic acid, polyvinyl alcohol, hydrophilic polyurethanes, poly hydroxyethylmethacrylate (HEMA), polyethyleneglycol(meth)acrylate, polyethoxypolyethyleneglycol(meth)acrylate, polymethoxyethyl(meth)acrylate, polyethoxy(meth) acrylate, poly 2-dimethylaminoethyl(meth)acrylate (DMAEMA) and poly 3-dimethylamino-propylmethacrylamid (DMAPMA), carbohydrates or gelatins.

The hydrophilic B block(s) in the amphiphilic block copolymers may also be prepared from different monomers, or oligomers, for example the monomer or oligomers used for the preparation of the above mentioned hydrophilic polymer blocks, or monomers or oligomers selected from acrylic acid, maleic acid, hydroxyethylmethacrylate (HEMA), vinylpyrrolidone (NVP), polyvinyl alcohol, polyethylene glycols and derivatives thereof, such as polyethyleneglycol-(meth)acrylate, ethoxypolyethyleneglycol(meth)acrylate, ethoxyethyl(meth)-acrylate, ethoxy(meth) acrylate, 2-dimethylamino-ethyl(meth)acrylate (DMAEMA) and 3-dimethylaminopropylmethacrylamid (DMAPMA), in order to impart desired properties (e.g Tg and modulus) into the polymer. Suitably, the hydrophilic block(s) may be a copolymer of neutralised (meth)acrylic acid, such as sodium acrylate and hydroxyethylmethacrylate (HEMA), vinylpyrrolidone (NVP), polyethyleneglycols and derivatives thereof, such as polyethylene-glycol(meth)acrylate and ethoxypolyethyleneglycol(meth)acrylate, methoxyethyl-(meth)acrylate, ethoxy(meth) acrylate, 2-dimethylamino-ethyl(meth)acrylate (DMAEMA) or 3-dimethylaminopropylmethacrylamid (DMAPMA).

As used herein (meth)acrylate means acrylate and methacrylate.

It is also possible to have a number of hydrophobic monomers, present in the hydrophilic B block(s), e.g. in order to impart desired properties into the polymer block(s), as long as the presence thereof provide a hydrophilic block that will be able to absorb significant amounts of water and is incompatible with the hydrophobic block(s) of the amphiphilic block copolymer(s).

Likewise, it is possible to have a number of hydrophilic monomers, present in the hydrophobic block(s), as long as the hydrophobic block(s) is incompatible with the hydrophilic block(s) of the amphiphilic block copolymer.

The hydrophilic B block(s) suitably has a minimum molecular weight of about 500 in order to be able to form separate hydrophilic domains in the coating composition.

Suitably, the hydrophilic B block(s) of the amphiphilic block copolymer has a molecular weight of at least 1000, preferably between 1000 and 300.000, more preferred between 50.000 and 300.000.

Preferably the molecular weight is higher than 1000 in case of end blocks and higher than 5000 in case of midblocks.

Preferably, hard hydrophilic B block(s) (i.e. polymers with high Tg) have a smaller size than softer hydrophilic B block(s) (i.e. blocks with a lower Tg).

The size or molecular weight of the hydrophobic A block(s) does not appear to be a limiting factor for the use of an amphiphilic block copolymer for the purpose of the present invention, as long as the hydrophobic block is sufficiently large to form a physical separation of the hydrophobic and hyd rophilic phases in the coating composition, i.e. hydrophobic domains or phases are formed.

Generally, the hydrophobic A block(s) of the amphiphilic block copolymer has a molecular weight of at least 1000, preferably between 1000 and 500.000, more preferred between 1000 and 300.000, more preferred between 1000 and 100. 000, or most preferred between 1000 and 50.000.

For hard hydrophobic A blocks, such as styrene blocks, the molecular weight of the block is typically between 10.000 and 20.000 and for soft blocks, such as butylacrylate, it is suitable between 50.000 and 100.000.

The amphiphilic block copolymers in the coating composition of the invention should maintain a high cohesion during and following swelling with a wetting agent according to the invention.

The use of amphiphilic block copolymers having long hydrophobic end blocks improves the cohesion in the coating dramatically.

A person skilled in the art will be able to select the appropriate size for the hydrophobic block(s) in view of the size of the hydrophilic block (s) and vice versa.

In one embodiment the hydrophobic A block in the amphiphilic block copolymer is a thermoplastic end block of a mono vinyl aromatic homo polymer, e.g. polystyrene.

In a particular embodiment, the amphiphilic block copolymer have at least two different molecular weight blocks in the copolymer (e.g., an A block of 1000 to 50,000 number average molecular weight and a B block of 1000 to 500,000 number average molecular weight), where the B domain is a hydrophilic end block polymer or the mid polymer block in case of a triblock copolymer.

Suitably the amphiphilic block copolymer is a triblock copolymer ABA having the hydrophilic block B as the midblock.

In one embodiment of the invention, the amphiphilic block copolymers for use in accordance with the present invention may be functionalised for further reactions like graft copolymerisation, cross-linking or for further polymerisation by inclusion of suitable functional groups.

The functional groups may be attached to the ends of the blocks or as side chains. The functional groups may e.g. be unsaturated vinyl groups containing double bonds for further polymerisation. The functional groups may furthermore be photo initiators attached to the block copolymers for UV-polymerisation for covalent cross-linking. The functional groups may be hydroxyl, primary or secondary amine groups for further reactions with isocyanate for the formation of polyurethane based block copolymers or for cross-linking with isocyanate.

Suitable amphiphilic block copolymers for the purpose of the present invention are a poly(styrene-b-acrylic acid-b-styrene) (such as P3000-SAAS with Mn 2000-65000-2000), poly(methyl methacrylate-b-methacrylic acid-b-methyl methacrylate) (such as P1483-MMAMAAMMA) and poly(styrene-b-ethylene oxide-b-styrene) (such as P2525-SEOS with Mn at 9500-48000-9500) all available from Polymer Source 124 Avro Street, Montreal, Quebec H9P 2X8, Canada as well as commercially available amphiphilic block copolymers from Rhodia and Atofina.

The amphiphilic block copolymers useful according to the invention may be prepared by a number of methods, such as free-radical polymerisation controlled by xantates, dithioesters, dithiocarbamates, iniferters, iodine degenerative transfer, tetraphenylethane derivatives, or organocobalt complexes, or by polymerisation using nitroxide precursors, atom transfer free-radical polymerisation (ATRP) and group transfer polymerisation. These methods with references are described in US Patent publication 2004/0030030.

An advantage of amphiphilic block copolymers which are cross linked by physical cross links, is that they are thermoplastic, which allow the coating composition to be heated and formed into articles e.g. by extrusion, injection moulding etc. and allows the coating composition to be applied on the medical device e.g. by co-extrusion. Both extrusions and co-extrusion are suitable ways of preparing tube formed medical devices, such as catheters.

When preparing the coating composition, the amphiphilic block copolymer is suitably added in an amount of from 10 to 100%, suitably 10-90 %, preferable 10-80 % or most preferred 10-50 % by weight of the total weight of the coating composition, i.e, before wetting with the wetting agent.

In addition to the amphiphilic polymer forming a physical cross-linked matrix in the coating composition, the coating composition may also contain other hydrophilic or hydrophobic polymer (s). This is also described in PCT/DK2004/000677 (published in WO 2005/033198) where it is suggested to include a hydrogel-forming hydrophilic homopolymer or heteropolymer that is compatible with the hydrophilic block of the amphiphilic block copolymer and incompatible with the hydrophobic blocks in the coating composition.

The hydrophilic homopolymer or heteropolymer may be the same type of polymer as those mentioned above for the hydrophilic B block.

The hydrophilic homopolymer or heteropolymer is suitably a cellulose derivative a polysaccharide, polyvinyl-pyrrolidone, polyvinyl alcohol, polycarboxylic acid polyacrylic acid, poly (methyl vinyl ether/ maleic anhydride), poly (meth)acrylic acid, polyethylenglycols (PEG), polyamides, polyacrylic amides and derivatives or blends of these polymers.

A suitable hydrophilic homopolymer or heteropolymer is a poly-vinylpyrrolidone polymer or a copolymer containing vinylpyrrolidone, such as ViviPrint 540, PVP K-90, PVP K-30, PVP K-25, PVP K-15, PVP K-12, and PVPNA S 630copolymer.

Other suitable hydrophilic homo- or heteropolymers are acrylic acids or copolymers containing acrylic acid, or hydrophilic copolymers of a poly (methyl vinyl ether/ maleic anhydride) or poly (methyl vinyl ether/ maleic-acid).

The ability of polymers based on acids, such as acrylic acid, to absorb water and transport water (water and vapour permeability) is highly pH dependent. Such polymers have to be neutralised with a base before use in order to obtain a polymer capable of absorbing and transporting sufficient amounts of water. Suitable, the pH should be around 7 in order to achieve the desired properties with such polymers.

Specific hydrogel forming hydrophilic polymers are the polymers selected from the group consisting of Luvicross® (a crosslinked and thus insoluble vinylpyrrolidone homopolymer), Luviskol® VA 37 E, Luviskol® VA 55 I VPNA Copolymer, Luviskol® VA 64 Powder and Luviskol® VA 73 E (all copolymers of vinylpyrrolidone and vinylacetate), Luvitec® VPI 55 K 72 W a vinylpyrrolidone/vinylimidazole and Luvitec® VPC 55 K 65 W (a vinylpyrrolidone/vinylcaprolactam copolymer), Luvicap® EG (a Polyvinylcaprolactam polymer from BASF), Luviset P.U.R. (a neutralized anionic polyurethane polymer), Luviquat Hold, Polyquatemium-46 (a copolymer of vinylcaprolactam (VCap), vinylpyrrolidone (VP) and quatemized vinylimidazole), Luviquat PQ 11 PN, Polyquaternium-11 (a quatemized copolymer of vinylpyrrolidone (VP) and dimethylaminoethylmethacrylate (DMAEMA), Luviquat UltraCare, Polyquaternium-44, Luviquat Care, Polyquatemium-44, Luviquat HM 552 Polyquaternium-16, Luviquat Style Polyquaternium-16, Luviquat FC 370 Polyquaternium-16, Luviquat FC 550 Polyquaternium-16 and Luviquat Excellence Polyquatemium-16 (all copolymers of vinylpyrrolidone (VP) and quatemized vinylimidazole), Polyquatemium-16 and Polyquaternium-44 (copolymers of vinylpyrrolidone (VP) and quatemized vinylimidazole (QVI), or Pluracare® F 68 NF, Poloxamer 188, Pluracare® F 87 NF, Poloxamer 237, Pluracare® F 108 NF Poloxamer 338, Pluracare® F 127 NF, Poloxamer 407, Pluracare® L 44 NF and Poloxamer 124 (all block copolymers and synthetic copolymers of ethylene oxide and propylene oxide) and polymethylvinylether.

Suitably, the coating composition comprises one or more polyethylene oxides having a molecular weight between 100.000 and 5. 000.000, such as Polyox WSR N-80 (Mw 200.000) and Polyox WSR 301 (Mw 4.000.000).

For the preparation of the coating composition of the invention, the hydrophilic homopolymers or heteropolymers are suitable added in an amount from 5 to '80%, preferably 10-80 % by weight of the total weight of the desired composition, i.e. before wetting.

In one embodiment of the invention the coating composition does not contain other hydrophilic or hydrophobic polymer (s) in addition to the amphiphilic polymer forming a physically cross linked matrix in the coating composition.

For some applications it is preferred that the coating composition comprises a plasticizer for the hydrophilic homopolymer or heteropolymer in order to ensure optimum elastic and plastic moduli for the intended use as a coating. This is especially the case when the hydrophilic homopolymer or heteropolymer is a polymer of a relatively high degree of polymerisation.

Suitable, the hydrophilic plasticizers for use in the coating compositions of the invention are selected from polyethylene glycol (e.g. PEG 300, PEG 400), propylene glycol, dipropylene glycol, glycerol, glycerol diacetate (diacetin), glycerol triacetate (triacetin), triethyl citrate (Citrofol Al), and acetyl methyl citrate (Citrofol All).

When using a plasticizer in the coating compositions of the invention, it is suitably selected from the group consisting of polyethylene glycols, such as PEG 400.

The lubricant contained in the wetting agent may be the same substance as the plasticizer used in the coating composition, however the lubricant is added with the wetting agent, whereas the plastizicer is added during manufacture of the coating composition

In one particular embodiment of the invention, the coating composition does not contain a hydrophilic plastizicer.

When preparing the coating composition of the invention, the plasticizer is suitably added in an amount of from 10 - 50 %m more suitably from 20 - 50% by weight of the total weight of the coating composition, i.e. before wetting with the wetting agent.

The compositions according to the invention may also comprise a cohesion-promoting component such as polyacrylic acid or polycarboxylic acid, acrylic acid copolymers or associative thickeners.

Associative thickeners are polymers that are based on water-soluble polymers capped with water insoluble hydrophobic groups. They may be acrylate polymers, cellulose ethers or, polyethylene-glycol capped with water-insoluble hydrophobic groups like fatty alcohols, for example. In water solution or in emulsion, these polymers form a network that increases the viscosity. The water-soluble backbone polymer is dissolved in water. The hydrophobic caps are adsorbed onto the hydrophobic polymers, or they form micelle structures with hydrophobic polymers. As each associative thickener polymer contains at least two hydrophobic caps, the result is a three-dimensional network within the emulsion. This increases the viscosity. Mainly the high- and mid-shear viscosity is affected.

Thickeners are suitably STABlLEZE® 06 & QM, GANTREZ®, polymethyl vinyl ether/maleic anhydride copolymers from ISP, Aculyn® 28 from Rohm and Haas. NEXTON® or Natrosol® Plus- CS both hydrophobically modified hydroxyethylcelluloses from HFRCULES®, or Carbopol homopolymer and copolymers such as Noveon AA1 and Pemulen TR 2 from Noveon.

Such cohesion-promoting component is suitably added to the coating composition in an amount of up to 15% by weight of the total weight of the composition prepared.

Still further, the coating compositions according to the invention may also comprise conventional used extenders or fillers such as polysaccharides like CMC, antioxidants, fibres, salts, clay, buffers, or pigments in an amount up to 30% of the total weight of the coating composition prepared.

The hydrophilic coating of the invention may also contain an osmolality increasing agent as described above, suitably a salt or urea.

The osmolality increasing salt may be an inorganic salt, such as sodium chloride, potassium chloride, potassium iodide, potassium nitrate, and organic salts such as sodium citrate or sodium benzoate.

The osmolality increasing salt or urea may form part of the coating composition or it may be a component in the wetting agent.

The appropriate amount of the different ingredients in the coating composition is best expressed by listing the amounts of the various ingredients used for the preparation of the coating composition. The amounts will more or less correspond to the amounts in the final coating composition although a minor variation in the amount of water (before wetting) may cause small or minor changes the percentages of the various ingredients in the composition. Thus, the coating composition suitably comprise:
10-100% w/w, or suitably 10-90 % w/w, preferably 10-50 % of an amphiphilic block copolymer,
at least 5 % w/w of a hydrophilic homopolymer or heteropolymer;
0-30 % w/w of a plastiziser for the hydrophobic phase
0-50 % w/w of a plastiziser for the hydrophilic phase.

In a particular embodiment of the invention, the coating composition comprises: 60 - 90 % w/w, suitably 60-80 % w/w or more preferred 70-80 % w/w of an amphilhilic polyurethane, 15-30 % w/w, suitably 15-25 % w/w, or more preferred 18-20 % w/w polyethylene oxide, and optionally up to 20 %, preferably up to 10 % of a plasticiser.

The amphiphilic polyurethane is typically selected from Tecogels, such as Tecogel 2000 and Tecogel 500, and Tecophilic 93A.

The polyethylene oxide is typically selected from Polyox WSR N-80 (Mw 200.000) and Polyox WSR 301 (Mw 4.000.000).

Typical recipes for coating compositions comprising amphiphilic polyurethane and polyethylene oxide are:
A) 75 % w/w Tecogel 2000
   24 % w/w Polyox WSR N-80
   1 % w/w Polyox WSR 301
B) 48 % w/w Tecogel 2000
   32 % w/w Tecophilic 93A
   20 % w/w Polyox WSR N-80
C) 43 % w/w Tecogel 2000
   29 % w/w Tecophilic 93A
   18 % w/w Polyox WSR N-80
   10 % w/w Santiziser S120 (plastiziser)
D) 40 % w/w Tecogel 2000
   40 % w/w Tecogel 500
   18 % w/w Polyox WSR N-180
   10 % w/w Santicizer S 120
   The coating compositions A) -D) may suitably be applied the the medical device in melted condition.
   Typical receipes for coating compositions comprising amphiphilic polyurethane and polyvinylpyrrolidone are
E) 50 % w/w PVP K-90
   50 % w/w Hydromed
F) 50 % w/w PVP K-25
   50 % w/w Tecogel 2000
G) 55 % w/w PVP K-90
   45 % w/w Hydroslip.
H) 35,3 % w/w PVP K-90
   27.9 % w/w Hydroslip
   36.8 % w/w Citrofol A1
I) 32.8 % w/w PVP K-25
   32.8 % w/w Hydroslip, Hydromed,or Tecogel 2000
   34.4 % w/w Citrofol A1
J) 33.9 % w/w PVP K-90
   26.9 % w/w Hydroslip
   3.9 % w/w Hydromed
   35.3 % w/w Citrofol A1
K) 39.3 % w/w PVP K-25
   26.2 % w/w Tecogel 500
   34.5% w/w Citrofol A1

The coating compositions E) - K) may suitably be applied to the medical device as a solution (e.g in dioxolane), followed by evaporation of the solvent and leaving the coating composition on the medical device.

The invention also relates to a method for the preparation of a medical device as described above comprising
a) providing a medical device having a coating comprising a hydrophilic polymer as described above;
b) placing the medical device or the part of the medical device carrying said coating in contact with the wetting agent comprising water and the lubricant and allowing the wetting agent to enter into the coating.

The medical device provided under step a) above may have an essentially dry coating before wetting. Alternatively the medical device provided under step a) has a more or less wetted coating.

The swelling of the coating composition may be carried out by dipping the hydrophilic coating in the wetting agent, e.g. a solution or emulsion comprising water and a lubricant.

The wetted hydrophilic coating of the invention may consist one or more layers The inner layer is suitably a binder binding the coating layer the medical device.

A person skilled in the art may determine a suitable amount of wetting agent in relation to the amount of hydrophilic coating composition.

Suitably, the percentage of wetting agent is above 50 % w/w , above 60% w/w, above 70 % w/w, above 85% w/w, 88% w/w, or above 90% w/w of the coating composition. Most suitable the percentage of wetting agent is above 85% w/w, more suitably above 88% w/w, or even more suitably above 90% w/w of the coating composition.

Suitably, the wetting agent comprises on or more lubricant(s) in a total amount between 0.1 % and 95 %, suitably between 0.1 % and 70%, or more suitably between 1 and 70 % by weight of the wetting agent. The optimal amount will depend on the specific composition of the coating composition and the lubricant(s).

When the hydrophilic polymer matrix comprises an amphiphilic polyurethane, such as Tecogel 2000, the hydrophilic polymer is polyethylene oxide and the lubricant is glycerol, the lubricant is suitably present in an amount between 0.1 % and 70 % by weight of the wetting agent.

Unless reference is made to the amount of lubricants in the wetting agent when the wetting agent is present in the coating composition, a reference to the amount of lubricant in the wetting agent, it is the amount of lubricant in the wetting agent, before it is used to wet the hydrophilic coating composition.

In cases where the coating composition itself contain one or more of the lubricants mentioned above, the wetting agent used to swell the coating may contain a smaller amount of lubricant(s) than the amount present in the wetting agent when present In the coating composition after swelling the coating composition with the wetting agent.

However, suitably the lubricant is present in an amount between 0.1 % and 95 %, suitably between 0.1 % and 70%, or more suitably between 1 and 70 % by weight of the wetting agent, even when the wetting agent is present/absorbed in the coating composition.

Suitably, at least 0.01 mm of the outermost layer of the wetted hydrophilic coating comprises between 0.1 % and 95 %, suitably between 0.1 % and 70%, or more suitably between 1 and 70 % by weight of the lubricant(s) in the wetting agent.

Medical devices or parts thereof carrying the hydrophilic coating of the invention, may be formed from a variety of basic materials, such as plastic, metals, glass, ceramics, etc. Typical examples of plastic materials for medical devices are polymers such as polyurethanes and copolymers thereof, or polyether block amides such as Pebax^{™} or other polymer materials including polyvinylchloride, polyamide, styrene-ethylene/butylenes-styrene block copolymers (SEBS), styrene-isoprene-styrene block copolymers (SIS), styrene-ethylene/propylene-styrene block copolymers (SEPS), ethylene-vinyl-acetate copolymers(EVA), polyethylene (PE) metallocene-catalyzed polyethylene and copolymers of ethylene and propylene or mixtures thereof. Currently very relevant material are polyurethane or copolymers thereof, as well as polyvinylchloride (PVC). Suitably, the medical device or the element of the medical device having a hydrophilic coating is formed from a polyurethane or PVC.

The surface of the medical device, to which the hydrophilic wetted coating is applied, may be the full surface of the medical device or a part thereof. In one embodiment, a part of the surface may be masked with a film or the like providing a predetermined pattern for the hydrophilic coating on the surface.

In one embodiment of the invention, the whole medical device or the part thereof having a hydrophilic coating is made of the coating composition.

According to this embodiment of the invention, there is no separate step for application of a coating composition to the surface of the medical device or a part thereof.

According to this embodiment of the invention, the medical device for example a catheter, may be made by injection moulding of the coating composition as described in WO 03/002325. Extrusion is also a possibility.

According to another embodiment of the invention, the medical device or a part thereof having a hydrophilic coating and the coating composition is constructed from different materials or compositions.

According to this embodiment of the invention, the medical device or the element of the medical device having a hydrophilic coating may be formed from any of the materials mentioned above using conventional methods for making such items from such materials and the coating composition is thereafter applied to the medical device or a part thereof. Application of the coating composition may be carried out by known techniques. However, where the coating composition is a thermoplastic material coating is suitably carried out by co-extrusion.

Injection moulding of more than one layer may be used for coating of a medical device with a coating composition of the invention. Co-extrusion also represents a possiblility.

The coating on the medical devices as mentioned above may also be applied to the surface thereof using conventional techniques, such as dipping in a coating solution.

The coating may have one or more layers. One layer may be a primer layer useful for attaching the coating composition to the medical device.

According to a preferred embodiment of the invention, the medical device according to the invention is a catheter, suitably a urinary catheter.

The medical device according to the invention may suitably be sterilised as described in WO 00/30696 or by analogous methods.

The medical device is suitably stored in a package, suitably a closed package designed so that storage of the medical device does not alter the characteristics of the hydrophilic coating. Suitably, the medical device or the part thereof carrying the hydrophilic coating is contained in a container impermeable to water and vapour.

Thus, the invention also relates to a package comprising a medical device as above wherein the medical device or the part of the medical device carrying said wetted hydrophilic coating is enclosed in a water and vapour impermeable container.

In one embodiment, the water and vapour impermeable container may contain additional water, wetting agent comprising lubricant, or another aqueous solution (e.g. saline), or a water vapour generating composition.

According to another embodiment of the invention, the water and vapour impermeable container does not contain additional water, wetting agent comprising lubricant, or another aqueous solution (e.g. saline), or a water vapour generating composition. According to this embodiment of the invention, the water and vapour impermeable container only contains the medical device or the part thereof carrying the wetted hydrophilic coating.

In one embodiment of the invention, the medical device carrying the coating composition is stored in a closed package containing a wetting agent comprising a lubricant.

Thus, the invention also relates to a package comprising a medical device having a hydrophilic coating comprising a coating composition as above and a wetting agent comprising one or more lubricant(s) where the coating and the wetting agent are kept separated until just before use.

Further embodiments of the invention are defined by claims 3 to 16, 17 and 18-21.

### Experimental section

### Example 1

Wafers made of pure Tecogel 2000 measuring 26 mm in diameter was tested according to the procedure described below:
1) A wafer is weighed (+/-0,001g) and is placed in a plastic container with a surplus of wetting agent and the plastic container is closed.
2) After about 24 hours the wetted wafer is removed from the plastic container and surplus of wetting agent is allowed to drip off (5-15 sec). The wetted wafer is then placed in a petri dish and covered with the lid. The plastic container with the wetting agent is closed and kept for later analysis.
3) The wetted wafer is weighed (+/-0,001g) and the friction is measured (fiddle method, see example 3) immediately thereafter (at 0 hours).
4) The wetted is again placed in the petri dish and stored at room temperature.
5) The steps under item 3) and 4) is repeated until the coating collapses or the friction measured is above 1000 mN.

The friction is measured three times for each combination of wafer and composition of the wetting agent (1 %, 8 %, 15 % and 30 % w/w of glycerol in 0.9 % w/w saline). Three identical wafers are tested each time.

Friction is measured at 0, 4, 24, 48 hours.

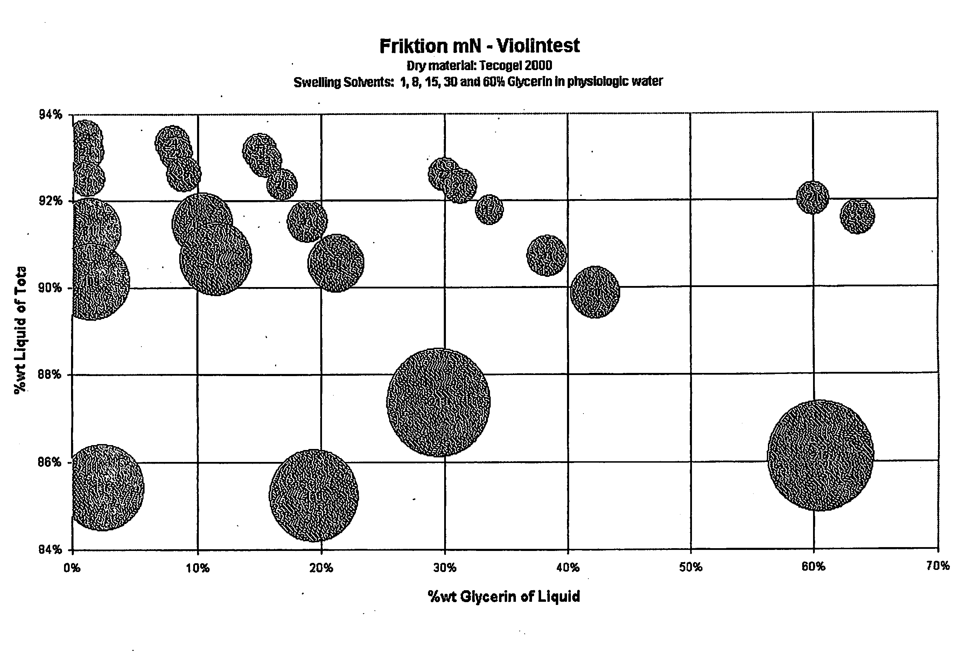

The results obtained are illustrated in figure 1.

As may be seen from the figures, the wetted wafers have a low friction when the percentage of wetting agent is above 89% w/w of the total weight of the wafer. The figures in the spheres is the friction measured in mN.

### Example 2

Smudging from the wafers was measured as follows:
A co-extruded hose was allowed to swell in a surplus of wetting agent for 24 hours and then allowed to drip off (15 sec). Smudging is measured by wiping with a paper tissue.

SpeediCath® (from Coloplast) is swollen with 10 ml PVP in water for at least 24 hours and then allowed to drip off (15 sec). Smudging is measured by scraping the wetted coating.

The results obtained are presented in table 1:

**Tablet 1: Excess of material on the surface of wetted products, RSC =Relative Standard deviation**

| Product | mg material wiped or scraped off/cm². | RSD |
|---|---|---|
| Co-extruded hose with 0.13 mm Tecogel 2000 | 3,2 | 13% |
| SpeediCath® Catheter (PVP coating) from Coloplast | 13.1 | 4% |

From the results it appears that smudging is lower with the amphiphilic-wetted materials than with the catheter coated with covalently cross linked PVP and swelled in a mixture of PVP and water.

### Example 3 The Fidle friction test

### Apparatus and material:

A friction apparatus with a Chatillon loadcelle (velocity 4 mm/s),
Velcroband 30x20 mm,
Metalblock 10x50x25 mm,
A tube covered with stainless steel (316), 32 mm long, diameter 4mm and having a total weight of 25.8g.

### Method:

The wafer is removed from the petri dish and cut into a size that fits the Velcro band. The metal tube is placed so that it lies on the wafer and the friction test is started. The wafer is moved 47 mm in one direction and 47 mm in the other direction.

## Claims

1. A medical device having a wetted hydrophilic coating comprising
a) a coating composition containing a hydrophilic polymer and
b) a wetting agent comprising water and one or more lubricant(s).

2. The medical device according to claim 1, wherein the hydrophilic polymer is an amphiphilic block copolymer containing hydrophilic and hydrophobic blocks.

3. The medical device according to claims 1-2, wherein the hydrophilic coating composition is thermoplastic.

4. The medical device according to any of claims 2-3, wherein the amphiphilic block copolymer is a polyurethane.

5. The medical device according to any of claims 2-4, wherein the coating composition comprises a hydrophilic hetero- or homopolymer.

6. The medical device according to claim 5, wherein the coating composition comprises a hydrophilic hetero- or homopolymer, which is compatible with the hydrophilic block(s) of the amphiphilic block copolymer and incompatible with the hydrophobic blocks of the amphiphilic block copolymer.

7. The medical device according to any of claims 5-6, wherein the hydrophilic homopolymer is polyethylene oxide.

8. The medical device according to any of claims 1-7, wherein the lubricant is a hydrophilic lubricant.

9. The medical device according to any of claims 1-7, wherein the lubricant is selected from the group consisting of glycerol, ethylene glycol, diethylene glycol, triethylenglycol, propylene glycol, dipropylene glycol, and 1,5-pentanediol.

10. The medical device according to any of claims 1-7, wherein the lubricant is a glycol ester.

11. The medical device according to claim 10, wherein the lubricant is selected from the group consisting of glycerol diacetate (diacetin) and glycerol triacetate (triacetin).

12. The medical device according to claim 8, wherein the lubricant is glycerol.

13. The medical device according to any of claims 1-12, wherein the wetting agent or the coating composition comprises an osmolality increasing agent.

14. The medical device according to any of claims 1-12, wherein the wetting agent or the coating composition comprises an osmolality increasing agent selected from the group consisting of salts and urea.

15. The medical device according to any of claims 1-14, wherein the amount of wetting agent is above 85% w/w.

16. The medical device according to any of claims 1-15 above, which is a catheter.

17. A method for the preparation of a medical device comprising the steps of:
a) providing a medical device having a coating composition containing the hydrophilic polymer;
b) the medical device or the part of the medical device carrying said coating composition is placed in contact with a wetting agent comprising water and one or more lubricants and the wetting agent is allowed to enter into the coating composition.

18. A package comprising a medical device according to any of claims 1-16, wherein the medical device or the part of the medical device carrying said wetted hydrophilic coating is enclosed in a water and vapour impermeable container.

19. A package according to claim 18, wherein the water and vapour impermeable container contains additional water, wetting agent comprising lubricant, or another aqueous liquid, or a water vapour generating composition.

20. A package according to claim 18, wherein the water and vapour impermeable container only contains the medical device or the part thereof carrying the wetted hydrophilic coating.

21. A package comprising a medical device having a hydrophilic coating of a coating composition as in any of claims 1-15 and a wetting agent comprising one or more iubricant(s) where the coating composition and the wetting agent are kept separated until just before use.

## Patentansprüche

1. Medizinische Vorrichtung, die eine befeuchtete hydrophile Beschichtung aufweist, die enthält:
a) eine Beschichtungszusammensetzung, die ein hydrophiles Polymer enthält, und
b) ein Befeuchtungsmittel, das Wasser und ein oder mehrere Schmiermittel umfasst.

2. Medizinische Vorrichtung nach Anspruch 1, bei der das hydrophile Polymer ein amphiphiles Blockcopolymer ist, das hydrophile und hydrophobe Blöcke enthält.

3. Medizinische Vorrichtung nach Anspruch 1 oder 2, bei der die hydrophile Beschichtungszusammensetzung thermoplastisch ist.

4. Medizinische Vorrichtung nach einem oder mehreren der Ansprüche 2 - 3, bei der das amphiphile Blockcopolymer ein Polyurethan ist.

5. Medizinische Vorrichtung nach einem oder mehreren der Ansprüche 2 - 4, bei der die Beschichtungszusammensetzung ein hydrophiles Heteropolymer oder Homopolymer enthält.

6. Medizinische Vorrichtung nach Anspruch 5, bei der die Beschichtungszusammensetzung ein hydrophiles Heteropolymer oder Homopolymer enthält, das mit dem hydrophilen Block oder den hydrophilen Blöcken des amphiphilen Blockcopolymers verträglich und mit den hydrophoben Blöcken des amphiphilen Blockcopolymers unverträglich ist.

7. Medizinische Vorrichtung nach einem der Ansprüche 5 - 6, bei der das hydrophile Homopolymer Polyethylenoxid ist.

8. Medizinische Vorrichtung nach einem oder mehreren der Ansprüche 1 - 7, bei der das Schmiermittel ein hydrophiles Schmiermittel ist.

9. Medizinische Vorrichtung nach einem oder mehreren der Ansprüche 1 - 7, bei der das Schmiermittel aus der Gruppe ausgewählt ist, die besteht aus Glycerin, Ethylenglycol, Diethylenglycol, Triethylenglycol, Propylenglycol, Dipropylenglycol und 1,5-Pentandiol.

10. Medizinische Vorrichtung nach einem oder mehreren der Ansprüche 1 - 7, bei der das Schmiermittel ein Glycolester ist.

11. Medizinische Vorrichtung nach Anspruch 10, bei der das Schmiermittel aus der Gruppe ausgewählt ist, die aus Glycerindiacetat (Diacetin) und Glycerintriacetat (Triacetin) besteht.

12. Medizinische Vorrichtung nach Anspruch 8, bei der das Schmiermittel Glycerin ist.

13. Medizinische Vorrichtung nach einem oder mehreren der Ansprüche 1 - 12, bei der das Befeuchtungsmittel oder die Beschichtungszusammensetzung ein die Osmolalität erhöhendes Mittel enthält.

14. Medizinische Vorrichtung nach einem oder mehreren der Ansprüche 1 - 12, bei der das Befeuchtungsmittel oder die Beschichtungszusammensetzung ein die Osmolalität erhöhendes Mittel enthält, das aus der Gruppe ausgewählt ist, die aus Salzen und Harnstoff besteht.

15. Medizinische Vorrichtung nach einem oder mehreren der Ansprüche 1 - 14, bei der die Menge des Befeuchtungsmittels mehr als 85 Gew.-% beträgt.

16. Medizinische Vorrichtung nach einem oder mehreren der obigen Ansprüche 1 - 15, bei der es sich um einen Katheter handelt.

17. Verfahren zur Herstellung einer medizinischen Vorrichtung, das folgende Schritte umfasst:
a) Versehen einer medizinischen Vorrichtung mit einer Beschichtungszusammensetzung, die das hydrophile Polymer enthält;
b) Inkontaktbringen der medizinischen Vorrichtung oder des Teils der medizinischen Vorrichtung, der die Beschichtungszusammensetzung trägt, mit einem Befeuchtungsmittel, das Wasser und ein oder mehrere Schmiermittel umfasst, und Eindringenlassen des Befeuchtungsmittels in die Beschichtungszuammensetzung.

18. Packung, die eine medizinische Vorrichtung nach einem der Ansprüche 1 - 16 enthält, wobei die medizinische Vorrichtung oder der Teil der medizinischen Vorrichtung, der die befeuchtete hydrophile Beschichtung trägt, in einem für Wasser und Dampf undurchlässigen Behälter eingeschlossen ist.

19. Packung nach Anspruch 18, bei welcher der für Wasser und Dampf undurchlässige Behälter zusätzliches Wasser, Befeuchtungsmittel, das Schmiermittel enthält, oder eine andere wässerige Flüssigkeit oder eine Wasserdampf erzeugende Zusammensetzung enthält.

20. Packung nach Anspruch 18, bei welcher der für Wasser und Dampf undurchlässige Behälter lediglich die medizinische Vorrichtung oder den Teil davon enthält, der die befeuchtete hydrophile Beschichtung trägt.

21. Packung, die eine medizinische Vorrichtung mit einer hydrophilen Beschichtung aus einer Beschichtungszusammensetzung wie in einem der Ansprüche 1 - 15 sowie ein Befeuchtungsmittel enthält, das ein oder mehrere Schmiermittel enthält, wobei die Beschichtungszusammensetzung und das Befeuchtungsmittel bis unmittelbar vor der Verwendung voneinander getrennt gehalten sind.

## Revendications

1. Dispositif médical comportant un revêtement hydrophile comprenant
a) une composition de revêtement contenant un polymère hydrophile et
b) un agent mouillant comprenant de l'eau et un ou plusieurs lubrifiant(s).

2. Le dispositif médical selon la revendication 1, dans lequel le polymère hydrophile est un copolymère à bloc amphiphile contenant des blocs hydrophiles et hydrophobes.

3. Le dispositif médical selon les revendications 1 à 2, dans lequel la composition de revêtement hydrophile est thermoplastique.

4. Le dispositif médical selon l'une quelconque des revendications 2 à 3, dans lequel le copolymère à bloc amphiphile est un polyuréthane.

5. Le dispositif médical selon l'une quelconque des revendications 2 à 4, dans lequel la composition de revêtement comprend un hétéro- ou homopolymère hydrophile.

6. Le dispositif médical selon la revendication 5, dans lequel la composition de revêtement comprend un polymère hétéro- ou homopolymère hydrophile, qui est compatible avec le ou les blocs hydrophiles du copolymère à bloc amphiphile et est incompatible avec les blocs hydrophobes du copolymère à bloc amphiphile.

7. Le dispositif médical selon l'une quelconque des revendications 5 à 6, dans lequel l'homopolymère hydrophile est l'oxyde de polyéthylène.

8. Le dispositif médical selon l'une quelconque des revendications 1 à 7, dans lequel le lubrifiant est un lubrifiant hydrophile.

9. Le dispositif médical selon l'une quelconque des revendications 1 à 7, dans lequel le lubrifiant est choisi dans le groupe constitué par le glycérol, l'éthylène glycol, le diéthylène glycol, le triéthylène glycol, le propylène glycol, le dipropylène glycol et le 1,5-pentanediol.

10. Le dispositif médical selon l'une quelconque des revendications 1 à 7, dans lequel le lubrifiant est un ester de glycol.

11. Le dispositif médical selon la revendication 10, dans lequel le lubrifiant est choisi dans le groupe constitué par le diacétate de glycérol (diacétine) et le triacétate de glycérol (triacétine).

12. Le dispositif médical selon la revendication 8, dans lequel le lubrifiant est le glycérol.

13. Le dispositif médical selon l'une quelconque des revendications 1 à 12, dans lequel l'agent mouillant ou la composition de revêtement comprend un agent augmentant l'osmolalité.

14. Le dispositif médical selon l'une quelconque des revendications 1 à 12, dans lequel l'agent mouillant ou la composition de revêtement comprend un agent augmentant l'osmolalité choisi dans le groupe constitué par des sels et de l'urée.

15. Le dispositif médical selon l'une quelconque des revendications 1 à 14, dans lequel la quantité d'agent mouillant est supérieure à 85 % en poids/poids.

16. Le dispositif médical selon l'une quelconque des revendications 1 à 15 ci-dessus, qui est un cathéter.

17. Procédé de préparation d'un dispositif médical, comprenant les étapes consistant à :
a) fournir un dispositif médical ayant une composition de revêtement comprenant le polymère hydrophile ;
b) placer le dispositif médical ou la partie du dispositif médical portant ladite composition de revêtement en contact avec un agent mouillant comprenant de l'eau et un ou plusieurs lubrifiant(s) et laisser l'agent mouillant pénétrer dans la composition de revêtement.

18. Emballage comprenant un dispositif médical selon l'une quelconque des revendications 1 à 16, dans lequel le dispositif médical ou la partie du dispositif médical portant ledit revêtement hydrophile mouillé est enfermé dans un conteneur imperméable à l'eau et à la vapeur.

19. Emballage selon la revendication 18, dans lequel le conteneur imperméable à l'eau et à la vapeur contient de l'eau additionnelle, un agent mouillant comprenant un lubrifiant, ou un autre liquide aqueux, ou une composition générant de la vapeur d'eau.

20. Emballage selon la revendication 18, dans lequel le conteneur imperméable à l'eau et à la vapeur contient seulement le dispositif médical ou une partie de celui-ci portant le revêtement hydrophile mouillé.

21. Emballage comprenant un dispositif médical ayant un revêtement hydrophile d'une composition de revêtement comme dans l'une quelconque des revendications 1 à 15 et un agent mouillant comprenant un ou plusieurs lubrifiant(s), où la composition de revêtement et l'agent mouillant sont maintenus séparés jusqu'au moment de leur utilisation.
